(19) Europäisches Patentamt / European Patent Office / Office européen des brevets

(11) **EP 4 722 374 A1**

(12) **EUROPEAN PATENT APPLICATION**
published in accordance with Art. 153(4) EPC

(43) Date of publication:
08.04.2026 Bulletin 2026/15

(21) Application number: 25749237.1

(22) Date of filing: 31.01.2025

(51) International Patent Classification (IPC):
*C12P 7/52* (2006.01)    *C12N 9/88* (2006.01)
*C12N 9/02* (2006.01)

(52) Cooperative Patent Classification (CPC):
C12N 9/0004; C12N 9/88; C12P 7/52

(86) International application number:
PCT/KR2025/099165

(87) International publication number:
WO 2025/165198 (07.08.2025 Gazette 2025/32)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.01.2024 KR 20240015266

(71) Applicant: LG Chem, Ltd.
Seoul 07336 (KR)

(72) Inventors:
• MOON, Sekwon
  Daejeon 34122 (KR)
• PARK, Jeyun
  Daejeon 34122 (KR)
• KIM, Jae Hyung
  Daejeon 34122 (KR)

(74) Representative: Plasseraud IP
104 Rue de Richelieu
CS92104
75080 Paris Cedex 02 (FR)

(54) **METHOD FOR PRODUCING 3-HYDROXYPROPIONIC ACID**

(57) The present invention relates to a method for preparing 3-HP and/or improving productivity of 3-HP by culturing cells at a high concentration and adjusting the concentration of initial glycerol and the concentration of additional glycerol and the addition time, while producing and culturing this high-concentration cell culture solution by fed-batch culture, and can produce a high concentration of 3HP from a high concentration of glycerol.

【FIG. 2】

EP 4 722 374 A1

**Description**

[TECHNICAL FIELD]

Cross-reference to related applications

The present application claims the benefit of the priority based on Korean Patent Application No. 10-2024-0015266 filed on January 31, 2024, and the entire contents disclosed in the documents of the corresponding Korean patent application are incorporated as a part in the present description.

The present application relates to a method for preparing 3-hydroxypropionic acid (hereinafter, also referred to as "3-HP" ) in high concentration and high productivity and/or a method for improving productivity of 3-HP, and more specifically, relates to a method for preparing 3-HP and/or improving productivity of 3-HP, which enable to produce a high concentration of 3-HP from a high concentration of glycerol in high productivity, while stably maintaining the activity of cells having 3-HP production ability.

[BACKGROUND ART]

3-hydroxypropionic acid (hereinafter, also referred to as "3-HP" ) is a platform compound that can be converted into various chemical substances such as acrylic acid, methyl acrylate, acrylamide, and the like. Since being selected as a Top 12 value-added bio-chemical from US Department of Energy (DOE) in 2004, it has been actively studied in the academic world and in the industry.

Production of 3-HP is achieved by largely two methods which are a chemical method and a biological method, but most of them are petrochemical products. The chemical method is non-environmentally friendly because initial materials are expensive and toxic substances are produced during the production process, and the like, and there is a concern about the depletion of petroleum resources, and therefore, when 3-HP is produced with biomass, it can reduce side effects of petrochemical products, whereas it can be used as a substitute for petroleum resources that are on the brink of being depleted.

Accordingly, research on biotechnological production of 3-HP has been developed, but due to low yield and productivity, despite of the possibility to produce 3-HP, continuous research on strain development and production process development is still needed.

[DISCLOSURE]

[TECHNICAL PROBLEM]

Accordingly, the present inventors have developed a fermentation technology that can increase the 3-HP production concentration by completely converting maximum glycerol efficiently for commercialization of 3-HP.

One embodiment of the present application provides a method for preparation of 3-hydroxypropionic acid (3-HP) and/or a method for improving productivity of 3-HP, comprising high-concentration cell culturing strains having productivity of 3-hydroxypropionic acid (3-HP) in a culture medium; and producing (converting) 3-HP by transferring the culture solution of the above step into a production medium comprising a substrate, and the producing is performed by a fed-batch process, and the production medium comprises glycerol, and additional glycerol is added to the production medium during producing.

[TECHNIAL SOLUTION]

The present application provides a method for preparation of 3-HP and/or a method for improving productivity of 3-HP, comprising high-concentration cell culturing strains having productivity of 3-hydroxypropionic acid (3-HP) in a culture medium, and then transferring the cultured culture solution into a production medium comprising a substrate to produce (convert) 3-HP, wherein the method converts 3-HP in high concentration and high productivity under the substrate inhibition concentration and maximal production rate conditions by consuming all glycerol by a fed-batch process according to the initial conditions having the substrate inhibition concentration and maximal production conditions and additional glycerol supply and supply time conditions slowly consuming glycerol.

More specifically, the method, relates to a method for preparation of 3-HP and/or a method for improvement of productivity of 3-HP, comprising the following steps:

(1) high-concentration cell culturing strains having productivity of 3-hydroxypropionic acid (3-HP) into a culture medium; and

(2) producing (converting) 3-HP by transferring the culture solution of the step (1) into a production medium comprising a substrate,

and the method may be characterized in that the production of 3-HP of the step (2) is performed by a fed-batch process, and

the production medium comprises glycerol, and additional glycerol is added to the production medium during production.

Hereinafter, the present invention will be described in more detail.

In the step (1) of the method for preparation of 3-HP and/or method for improving productivity of 3-HP provided in the present description, cells having productivity of 3-hydroxypropionic acid are high-concentration cultured in a growth medium. After the high-concentration culture, the resulting culture may be directly used in step (2) to produce 3-HP without any separate cell recovery step. Specifically, the culture solution obtained from step (1) may be transferred to a production medium without undergoing an additional step of cell recovery.

In the step (1), the culture medium may not comprise glycerol as a carbon source.

In the present description, the 3-hydroxypropionic acid producing cell (hereinafter, interchangeably used with the same meaning as 'cell having productivity of 3-hydroxypropionic acid' ) may be selected from microorganisms which can produce 3-HP from a carbon source (for example, glycerol) in a production medium, for example, microorganisms consisting of microorganisms of the genus *Escherichia* (*E. coli*, etc.), the genus Pseudomonas, the genus Enterobacteria, the genus Brevibacterium, the genus Corynebacterium, the genus Klebsiella, the genus Citrobacter, the genus Clostridium, the genus Streptomyces, the genus Bacillus, the genus Lactobacillus, the genus Pseudomonas, the genus Saccharomyces and the genus Aspergillus, but not limited thereto. In one specific embodiment, the 3-hydroxypropionic acid producing cell may be *E. coli.*

In one embodiment, the 3-hydroxypropionic acid producing cell may comprise a gene encoding at least one (for example, 1 or 2 all) selected from the group consisting of glycerol dehydratase and aldehyde dehydrogenase. In one embodiment, the 3-HP producing cell may further comprise a gene (gdrAB) encoding glycerol dehydratase reactivase (GdrAB) additionally. In one embodiment, the 3-HP producing cell may a cell additionally capable of biosynthesizing vitamin B12.

The glycerol dehydratase may be encoded by dhaB (GenBank accession no. U30903.1) gene, but not limited thereto. The dhaB gene may be an enzyme derived from Klebsiella pneumonia, but not limited thereto. The gene encoding glycerol dehydratase may comprise a gene encoding dhaB1, dhaB2 and/or dhaB3. The glycerol dehydratase protein and a gene encoding thereof may comprise a mutation of the gene and/or amino acid sequence within a range that maintains enzyme activity of degrading glycerol to 3-hydroxypropanal (3-HPA) and water ($H_2O$).

The gene encoding aldehyde dehydrogenase (ALDH) (aldH), may be, for example, aldH (GenBank Accession no. U00096.3; EaldH) gene derived from an Escherichia coli or E. coli K12 MG1655 cell line, puuC gene derived from Klebsiella pneumonia (K. pneumonia), an/or KGSADH gene derived from Azospirillum brasilense, but not limited thereto. The aldehyde dehydrogenase protein and gene encoding thereof may comprise a mutation of the gene and/or amino acid sequence within a range that maintains activity to produce 3-HP from 3-HPA.

The 3-HP producing cell may comprise a gene encoding at least one, at least two, or all the 3 kinds of proteins selected from the group consisting of glycerol dehydratase, aldehyde dehydrogenase, and glycerol dehydratase reactivase, or a recombinant vector comprising the gene.

The recombinant vector may be used by replacing a promoter and a regulatory site within a range of the purpose to express a gene encoding at least one, at least two, or all the 3 kinds of proteins selected from the group consisting of glycerol dehydratase, aldehyde dehydrogenase, and glycerol dehydratase reactivase in a cell by a method known in the art.

The high-concentration culturing may be performed using a method known in the art without limitation within a range of the purpose to secure the 3-HP producing cells in large quantities, and in one embodiment, the culturing may be performed by fed-batch culture.

In one embodiment, the fed-batch culture may be performed by pH-stat method, DO-stat feeding method, continuous feeding method, or a combination thereof. In one embodiment, when the fed-batch culture using the pH-stat method is performed, glucose may be added at a concentration of 1 to 5g/L, but not limited thereto. In one embodiment, when the fed-batch culture in the continuous feeding method is performed, glucose may be added at a rate of 7 to 21 g/L/h, but not limited thereto.

According to one embodiment, the pH of the culture solution may be adjusted by adding a compound such as ammonium hydroxide, potassium hydroxide, ammonia, phosphate and sulfate into a microorganism culture solution in an appropriate manner during culturing. In one embodiment, during high-concentration culture, the pH may be maintained at 5 to 7.5, 5 to 7, 5.5 to 7.5, 5.5 to 7, 6 to 7.5 or 6.5 to 6, but not limited thereto.

In one embodiment, during the high-concentration culturing, the carbon source may be used by selecting monosaccharides, disaccharides, and/or polysaccharides within a range of the purpose for high-concentration culture without limitation. For example, the carbon source may be at least one, two or more, three or more, four or more, five or more, 10 or more, or all the 11 kinds selected from the group consisting of glucose, fructose, galactose, mannose, arabinose, xylose, ribose, sucrose, maltose, lactose, and cellobiose. The medium used during high-concentration culturing may not comprise glycerol as the carbon source. In this way, as the medium used during high-concentration culturing does not comprise glycerol, 3-HP production does not occur in the step of high-concentration culturing.

In one embodiment, the cell concentration after high-concentration culturing may be 10 or more, 30 or more, 50 or more, 70 or more, 100 or more, or 110 or more based on the $OD_{600}$ value, and for example, when culturing is performed for 20 hours, the $OD_{600}$ value may be 10 to 500, 10 to 400, 10 to 300, 10 to 250, 10 to 200, 10 to 150, 30 to 500, 30 to 400, 30 to 300, 30 to 250, 30 to 200, 30 to 150, 50 to 500, 50 to 400, 50 to 300, 50 to 250, 50 to 200, 50 to 150, 70 to 500, 70 to 400, 70 to 300, 70 to 250, 70 to 200, 70 to 150, 100 to 500, 100 to 400, 100 to 300, 100 to 250, 100 to 200, 100 to 150, 110 to 500, 110 to 400, 110 to 300, 110 to 250, 110 to 200, or 110 to 150, but not limited thereto.

In one embodiment, the cell concentration after high-concentration culturing may be 10 to 100g/L, 10 to 80g/L, 10 to 70g/L, 10 to 60g/L, 10 to 55g/L, 20 to 100g/L, 20 to 80g/L, 20 to 70g/L, 20 to 60g/L, 20 to 55g/L, 30 to 100g/L, 30 to 80g/L, 30 to 70g/L, 30 to 60g/L, 30 to 55g/L, 40 to 100g/L, 40 to 80g/L, 40 to 70g/L, 40 to 60g/L, 40 to 55g/L, 45 to 100g/L, 45 to 80g/L, 45 to 70g/L, 45 to 60g/L, or 45 to 55g/L, for example, 50g/L, based on the cell dry weight (g) per 1L of medium, but not limited thereto.

According to one embodiment, the pH of the culture solution may be adjusted by adding a compound such as ammonium hydroxide, potassium hydroxide, ammonia, phosphate and sulfate into a microorganism culture solution in an appropriate manner during culturing. In one embodiment, during high-concentration culture, the pH may be maintained at 5 to 7.5, 5 to 7, 5.5 to 7.5, 5.5 to 7, 6 to 7.5 or 6.5 to 6, but not limited thereto.

The temperature of the culture solution may be 20°C to 45°C, 25°C to 40°C or 30°C to 37°C, and for example, it may be 37°C.

In the present invention, for high-concentration cell culture, the 3-HP producing cells may be generated at a concentration of $OD_{600}$ per hour (optical density (O.D.) measured at 600nm) of 20 or less, 2 or more.

In one embodiment, the cell concentration after the high-concentration culture may be $OD_{600}$ 10 or more, 50 or more, 100 or more, 150 or more, 200 or more, 10 to 500, 10 to 400, 10 to 300, 10 to 250, 50 to 500, 50 to 400, 50 to 300, 50 to 250, 100 to 500, 100 to 400, 100 to 300, 100 to 250, 150 to 500, 150 to 400, 150 to 300, 150 to 250, 200 to 500, 200 to 400, 200 to 300, or 200 to 250, but not limited thereto. Specifically, the cell concentration may be 100 to 200 at $OD_{600}$.

According to one embodiment, the medium may comprise a nitrogen source and a trace element component, with the carbon source. The available nitrogen source may include peptone, yeast extract, meat juice, malt extract, corn steep liquor, soybean meal and urea or inorganic compounds, for example, ammonium sulfate, ammonium chloride, ammonium phosphate, ammonium carbonate and ammonium nitrate. The nitrogen source may be also used individually or as a mixture, but not limited thereto. The available phosphorus source may include potassium dihydrogen phosphate or dipotassium hydrogen phosphate or sodium-containing salts corresponding thereto, but not limited thereto. In addition, the medium may contain a metal salt such as magnesium sulfate or iron sulfate required for growth, but not limited thereto. In addition, essential growth substances such as amino acids and vitamins may be included. Furthermore, precursors suitable for the medium may be used. The medium or individual component may be added to the culture solution in a fed-batch method or in a continuous method by an appropriate method during the culturing process, but not limited thereto.

The step (2) is a step of producing (converting) 3-HP by transferring the culture solution of the step (1) into a production medium comprising a substrate, and is a step performed by a fed-batch process, in which then, the production medium comprises glycerol, and additional glycerol is added to the production medium by a fed-batch culture process.

The substrate may be glucose and/or glycerol, but not limited thereto. Specifically, the step (2) is a step of producing 3-HP by a fed-batch process under the conditions where the initial glycerol concentration and the addition time and rate of additional glycerol, by inoculating cells having productivity of 3-hydroxypropionic acid (3-HP) into a 3-hydroxypropionic acid production medium and transferring into the production medium.

The 3-hydroxypropionic acid production medium may be used without limitation within a range of the purpose of enabling the cells to produce 3-HP without causing proliferation (cell division, growth, or growth and development) of the 3-HP producing cells.

In one embodiment, the carbon source of the production medium may be glycerol, but not limited thereto. In one embodiment, the production medium may further comprise vitamin B12. In one embodiment, the production medium may not comprise glucose during cell inoculation.

In the present invention, it is characterized by overcoming a problem of inhibition of production by high-concentration glycerol by dividing and adding glycerol as a carbon source, and converting high-concentration 3-HP by consuming a maximal amount of glycerol.

Accordingly, the present invention is characterized in that the glycerol concentration (initial glycerol concentration) in the production medium at the beginning of the culturing of the step (2) is controlled. Specifically, the initial glycerol concentration may be 100 g/L to 150 g/L, 100 g/L to 145 g/L, 100 g/L to 140 g/L, 100 g/L to 135 g/L, 100 g/L to 130 g/L, 100 g/L to 125 g/L, 100 g/L to 120 g/L, 110 g/L to 150 g/L, 110 g/L to 145 g/L, 110 g/L to 140 g/L, 110 g/L to 135 g/L, 110 g/L to 130 g/L, 110 g/L to 125 g/L, 110 g/L to 120 g/L, 115 g/L to 150 g/L, 115 g/L to 145 g/L, 115 g/L to 140 g/L, 115 g/L to 135 g/L, 115 g/L to 130 g/L, 115 g/L to 125 g/L, 115 g/L to 120 g/L, 120 g/L to 150 g/L, 120 g/L to 145 g/L, 120 g/L to 140 g/L, 120 g/L to 135 g/L, 120 g/L to 130 g/L, 120 g/L to 125 g/L, 125 g/L to 150 g/L, 125 g/L to 145 g/L, 125 g/L to 140 g/L, 125 g/L to 135 g/L, 125 g/L to 130 g/L, 130 g/L to 150 g/L, 130 g/L to 145 g/L, 130 g/L to 140 g/L, 130 g/L to 135 g/L, 135 g/L to 150 g/L, 135 g/L to 145 g/L, 135 g/L to 140 g/L, 140 g/L to 150 g/L, 140 g/L to 145 g/L, or 145 g/L to 150 g/L, for example, 120, 125, 135, or 145 g/L. When the initial glycerol concentration is within the above range, it is preferable since 3-HP is produced at a high concentration and there is no residual glycerol.

In addition, in the step (2), it is characterized by adding additional glycerol to the production medium during 3-HP production. In one embodiment, the concentration of glycerol added additionally to the production medium may be an amount corresponding to 70 to 140g/L, 70 to 120g/L, 70 to 110g/L, 80 to 140g/L, 80 to 120g/L, 80 to 110g/L, 90 to 140g/L, 90 to 120g/L, or 90 to 110g/L, for example, 100g/L, based on the total volume of the production medium, but not limited thereto.

In one embodiment, the time of addition of the additionally supplied glycerol may be the time when the strains having productivity of 3-HP can produce 3-HP under the optimal conditions after production starts, and for example, it may be added at a uniform rate after 1 to 10 hours, 1 to 8 hours, 1 to 6 hours, 1 to 5 hours, 2 to 10 hours, 2 to 8 hours, 2 to 6 hours, 2 to 5 hours, 3 to 10 hours, 3 to 8 hours, 3 to 6 hours, or 3 to 5 hours, for example, 4 hours, but not limited thereto.

The additionally added glycerol may be supplied for 1 to 10 hours, 1 to 8 hours, 1 to 6 hours, 3 to 10 hours, 3 to 8 hours, 3 to 6 hours, 5 to 10 hours, 5 to 8 hours, or 5 to 6 hours, but not limited thereto.

In one embodiment, the medium may be synthetic media or semisynthetic media, but not limited thereto.

The cells for inoculation may be prepared in the form of a cell culture solution without a separate process of cell recovery after high-concentration culture of cells having productivity of 3-hydroxypropionic acid (3-HP) in the step (1), but not limited thereto.

The inoculation concentration of the high-concentration cultured cells (cell concentration during inoculation) may be appropriately adjusted or determined by those skilled in the art within the range of purpose to producing 3-HP. In one embodiment, the inoculation concentration (based on dry cell weight (DCW)/medium volume (L)) may be 1 to 20 g/L, 1 to 16 g/L, 1 to 12 g/L, 1 to 9 g/L, 2 to 20 g/L, 2 to 16 g/L, 2 to 12 g/L, 2 to 9 g/L, 4 to 20 g/L, 4 to 16 g/L, 4 to 12 g/L, or 4 to 9 g/L, but not limited thereto.

In the step of producing 3-HP, proliferation of the inoculated cells may not occur. In one embodiment, the number of the cells at the end of the step of producing may be 150% or less, 130% or less, 100% or less, 90% or less, or 80% or less, for example, 50 to 150%, 50 to 130%, 50 to 100%, 50 to 90%, 50 to 80%, 70 to 150%, 70 to 130%, 70 to 100%, 70 to 90%, or 70 to 80% of the number of the inoculated cells, but not limited thereto.

In one embodiment, by at least one selected from the group consisting of stirring rate control, air supply control, and pressure control and the like during culturing, DO (dissolved oxygen) may be adjusted. For example, to increase dissolved oxygen in a medium or culture, (1) at least one of the stirring rate, air supply and pressure may be increased, and (2) to reduce dissolved oxygen, dissolved oxygen in a medium or culture may be maintained in a certain range, by reducing at least one of the stirring rate, air supply and pressure, but not limited thereto.

According to one embodiment, to adjust the pH in the above range constantly, a compound selected from the group consisting of calcium hydroxide, magnesium hydroxide, ammonia hydroxide, sodium hydroxide and potassium hydroxide may be added to microorganism culture solution in an appropriate manner during culturing. Additionally, according to one embodiment, to maintain the temperature of the culture solution, a conventional method of temperature control may be used.

The culturing period may continue until the desired yield of a useful substance (for example, 3-HP) is obtained, and for example, it may be 3 to 60 hours, 3 to 48 hours, 3 to 36 hours, 3 to 28 hours, 6 to 60 hours, 6 to 48 hours, 6 to 36 hours, 6 to 28 hours, 12 to 60 hours, 12 to 48 hours, 12 to 36 hours, 12 to 28 hours, 20 to 60 hours, 20 to 48 hours, 20 to 36 hours or 20 to 28 hours, and for example, it may be 24 hours, but not limited thereto.

The 3-HP yield of the method for preparation of 3-HP and/or method for improvement of productivity of 3-HP provided in the present description may be for example, 80% or more, 85% or more, 90% or more, 93% or more, or 95% or more, but not limited thereto. The 3-HP yield may be calculated as the 3-HP production amount in the medium (culture) compared to the used amount (moles) of glycerol in the medium (production medium) in the step of producing 3-HP, and in one embodiment, it may be calculated by the following Equation 1.

Yield (%) = [(Final amount of 3-HP(g))/{(Glycerol before culturing (before producing 3-HP) (g)) - (Glycerol remaining after culturing (after producing 3-HP) (g))}] * 100     [Equation 1]

The 3-HP productivity of the method for preparation of 3-HP and/or method for improvement of productivity of 3-HP provided in the present invention may be 6.0 g/L/h or more, 6.5 g/L/h or more, 6.6 g/L/h or more, 6.0 to 60 g/L/h, 6.0 to 60 g/L/h, 6.0 to 20 g/L/h, 6.0 to 10 g/L/h, 6.5 to 60 g/L/h, 6.5 to 40 g/L/h, 6.5 to 20 g/L/h, 6.5 to 10 g/L/h, 6.6 to 60 g/L/h, 6.6 to 40 g/L/h, 6.6 to 20 g/L/h, or 6.6 to 10 g/L/h, for example, 6.68 g/L/h, but not limited thereto. The 3-HP production ability may be calculated by dividing the final 3-HP production concentration in the step of producing 3-HP by the total 3-HP production time, and in one embodiment, it may be calculated by the following Equation 2.

3-HP production ability = Final 3-HP production concentration (g/L) / Total 3-HP production time (h)     [Equation 1]

The 3-HP production amount of the method for preparation of 3-HP and/or method for improvement of productivity of 3-HP provided in the present description, may be for example, 100g/L or more, 110g/L or more, 120g/L or more, 130g/L or more, 140g/L or more, 145g/L or more, 100g/L to 1000g/L, 110g/L to 1000g/L, 120g/L to 1000g/L, 130g/L to 1000g/L, 140g/L to 1000g/L, or 145g/L to 1000g/L, based on the 3-HP content (g) per 1L of the medium during culturing (3-HP production) for 20 to 30 hours (for example, 20 hours, 21 hours, 22 hours, 23 hours, 24 hours, 25 hours, 26 hours, 27 hours, 28 hours, 29 hours, or 30 hours) (the upper limit may be selected from 130 to 1000g/L without special limitation, and for example, it may be 1000g/L, 500g/L, 400g/L, 300g/L, 200g/L, 160g/L or 147g/L, but not limited thereto).

The method for preparation of 3-HP and/or method for improvement of productivity of 3-HP provided in the present description may consume most of the glycerol added for 3-HP production. The remaining glycerol after the production may be produced at a concentration of 2 g/L or less, 1.8 g/L or less, 1.5 g/L or less, 1.3 or 1.0 g/L or less in the total culture solution (then, the lower limit of the by-product concentration may be selected from 0 to 0.000001g/L, but not limited thereto), or may be produced at a concentration of 0 g/L (the by-product is not produced at a detectable concentration).

The method for preparation of 3-HP and/or method for improvement of productivity of 3-HP provided in the present description may produce by-products resulting from 3-HP production at a low content. The by-products may be at least one selected from the group consisting of acetic acid, orotic acid, propionic acid, succinic acid, formic acid, uracilic acid and citric acid.

The high-concentration and high-production 3-HP preparation method provided in the present invention, may exhibit the 3-HP productivity (g/L/h) of 1.05 times or higher or 1.1 times or higher than the batch fermentation method, and for example, it may be 1.05 to 10 times, 1.05 to 5 times, 1.05 to 2 times, 1.05 to 1.7 times, 1.05 to 1.5 times, 1.05 to 1.2 times, 1.1 to 10 times, 1.1 to 5 times, 1.1 to 2 times, 1.1 to 1.7 times, 1.1 to 1.5 times or 1.1 to 1.3 times higher, and for example, it may be about 1.13 times higher.

Other embodiment provides a culture of 3-hydroxypropionic acid producing cells having a high 3-hydroxypropionic acid content and a low by-product content.

The culture may comprise 3-hydroxypropionic acid of 100g/L or more, 110g/L or more, 120g/L or more, 130g/L or more, 140g/L or more, 145g/L or more, 100g/L to 1000g/L, 110g/L to 1000g/L, 120g/L to 1000g/L, 130g/L to 1000g/L, 140g/L to 1000g/L, or 145g/L to 1000g/L, based on the total culture (the upper limit may be selected from 130 to 1000g/L without special limitation, and for example, it may be 1000g/L, 500g/L, 400g/L, 300g/L, 200g/L, 160g/L or 147 g/L, but not limited thereto). As one specific embodiment, the culture may comprise the by-products with 3-hydroxypropionic acid at a concentration of 100 g/L or more..

In addition, the culture may comprise acetic acid at a content of 0 to 1 g/L, 0 to 0.5 g/L, 0 to 0.3 g/L, 0.01 to 1 g/L, 0.01 to 0.5 g/L or 0.01 to 0.3 g/L, for example, 0.3 g/L, based on the total culture volume, but not limited thereto.

The culture may comprise orotic acid at a content of 0 to 0.5 g/L, 0 to 0.3 g/L, 0 to 0.2 g/L, 0 to 0.15 g/L, 0.01 to 0.5 g/L, 0.01 to 0.3 g/L, 0.01 to 0.2 g/L, or 0.01 to 0.15 g/L, for example, 0.14 g/L, based on the total culture volume, but not limited thereto.

The culture may comprise propionic acid at a content of 0 to 0.5 g/L, 0 to 0.3 g/L, 0 to 0.2 g/L, 0.01 to 0.5 g/L, 0.01 to 0.3 g/L, or 0.01 to 0.2 g/L, for example, 0.19 g/L, based on the total culture volume, but not limited thereto.

The culture may comprise succinic acid at a content of 0 to 1 g/L, 0 to 0.5 g/L, 0 to 0.3 g/L, 0 to 0.25 g/L, 0.01 to 1 g/L, 0.01 to 0.5 g/L, 0.01 to 0.3 g/L, or 0 to 0.3 g/L, for example, 0.2 g/L, based on the total culture volume, but not limited thereto.

The culture may comprise formic acid at a content of 0 to 0.5 g/L, 0 to 0.3 g/L, 0 to 0.2 g/L, 0 to 0.15 g/L, 0.01 to 0.5 g/L, 0.01 to 0.3 g/L, 0.01 to 0.2 g/L, or 0.01 to 0.15 g/L, for example, 0.14 g/L, based on the total culture volume, but not limited thereto.

The culture may comprise uracil acid at a content of 0 to 0.1 g/L, 0 to 0.05 g/L, 0 to 0.03 g/L, 0.01 to 0.1 g/L, 0.01 to 0.05 g/L, or 0.01 to 0.03 g/L, for example, 0.02 g/L, based on the total culture volume, but not limited thereto.

The culture may comprise citric acid at a content of 0 to 0.1 g/L, 0 to 0.05 g/L, 0 to 0.02 g/L, or 0 to 0.01 g/L, based on the total culture volume, but not limited thereto.

In one specific embodiment, the culture may be obtained by the method for preparation of 3-HP and/or method for improvement of productivity of 3-HP described above, and in the aspect of 3-HP preparation and productivity improvement, it may be advantageous to comprise by-products in the above content range.

In particular, in the culture, by-products may be comprised in the total culture having a high content of 3-HP produced during 3-HP production, and "orotic acid" is an intermediate of the pyrimidine biosynthesis pathway of a microorganism, and may be generated when 3-HP is produced by an aerobic microorganism.

The culture may comprise the by-products (for example, orotic acid), but it may be characterized by a low content of by-products, and for example, the culture produced by the method for preparation of 3-HP and/or method for improvement of productivity of 3-HP provided in the present description and/or the culture having a high content of 3-HP may have a lower content of by-products (for example, orotic acid), compared to a culture produced by a method for preparing 3-HP and/or a method for improving productivity of 3-HP in a different manner and/or a culture comprising 3-HP of different compositions.

As one specific embodiment, the culture may comprise by-products at the above content with 3-hydroxypropionic acid of 100 g/L or less, 200 g/L or less, or 300 g/L or less.

In one specific embodiment, the culture may be obtained by the aforementioned method for preparation of 3-HP and/or method for improvement of productivity of 3-HP, but not limited thereto.

In one specific embodiment, the culture may be obtained by the aforementioned method for preparation of 3-HP and/or method for improvement of productivity of 3-HP, but not limited thereto.

The culture may be used for a use in producing 3-hydroxypropionic acid.

Accordingly, other embodiment provides a composition for producing 3-hydroxypropionic acid comprising the culture.

Other embodiment provides a method for producing 3-hydroxypropionic acid, comprising separating, recovering, and/or purifying 3-hydroxypropionic acid from the composition for producing 3-hydroxypropionic acid.

[ADVANTAGEOUS EFFECTS]

The method for production and/or method for improving productivity of 3-HP provided in the present invention can produce 3-HP at a high concentration and high-productivity by applying a method for setting the fed-batch process and the initial concentration of glycerol and the concentration and time of additional addition, and the speed conditions during 3-HP production. By producing 3-HP in this high concentration and high productivity manner, it is possible to reduce investment costs for the same production volume. In particular, not only the cost of raw materials but also the operating costs for separation and purification can be reduced, thereby lowering the overall production cost.

[BRIEF DESCRIPTION OF THE DRAWINGS]

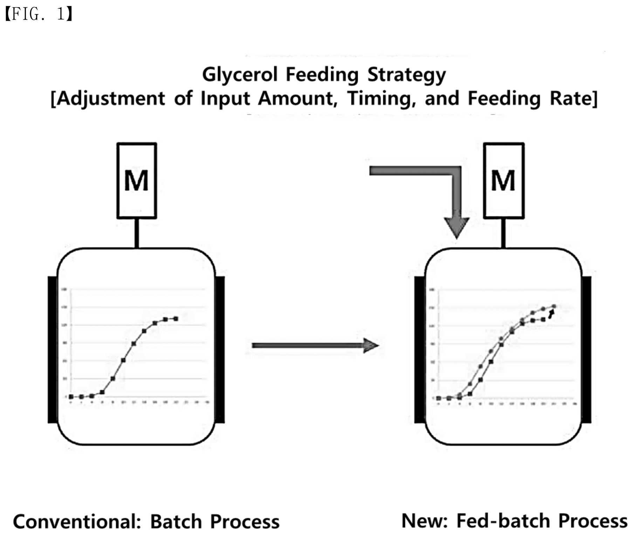

FIG. 1 is a schematic diagram showing the technical features of the present invention compared to the prior art.
FIG. 2 is a graph of the results measuring the production amount of 3-HP during 2-step culturing under the conditions of the control group (Comparative example 1) and Example 1.
FIG. 3 is a graph of the results measuring the production amount (g/L) of 3-HP depending on the initial glycerol concentration during 3-HP production.
FIG. 4 is a graph of the results measuring the production amount (g/L) of 3-HP depending on the concentration of initial and additionally added glycerol during 3-HP production.

[MODE FOR INVENTION]

Hereinafter, the present invention will be described by examples in more detail. However, the following examples are intended to illustrate the contents of the present invention only, but the scope of the present invention is not limited by the following examples.

In the present invention, unless otherwise mentioned, the temperature is based on Celsius temperature all, and nucleic acid sequences are written in the 5'-to-3' direction, unless otherwise specified.

**Reference Example 1. Preparation of 3-HP producing strain**

A 3-HP producing strain to be used for the method for preparation according to the present invention, was prepared according to the method disclosed in Korean Patent Application No. 10-2021-0151641, and was used for the following Comparative examples and Examples.

**Comparative example 1. Preparation of 3-HP by fed-batch culture during 2-step cell culture for 3-HP production**

Comparative example 1-1. High-concentration culture of cells (first step culturing)

The 3-HP producing strain prepared in Reference example 1 was under high-concentration cell culture with a 5L fermenter (Working volume 2L) by a fed-batch culture method.

Specifically, 20 g/L of glucose was separately added to an MR medium ($KH_2PO_4$ 6.67g, $(NH_4)_2HPO_4$ 4g, $MgSO_4 \cdot 7H_2O$ 0.8g, citric acid 0.8g, and trace metal solution 5mL per 1L; herein, Trace metal solution is 5M HCl 5mL, $FeSO_4 \cdot 7H_2O$ 10g, $CaCl_2$ 2g, $ZnSO_4 \cdot 7H_2O$ 2.2g, $MnSO_4 \cdot 4H_2O$ 0.5g, $CuSO_4 \cdot 5H_2O$ 1g, $(NH_4)_6Mo_7O_2 \cdot 4H_2O$ 0.1g, and $Na_2B_4O_2 \cdot 10H_2O$ 0.02g per 1L), and it was used as a cell culture medium, and the temperature of 35°C was maintained. The pH was maintained at 6.95 using ammonia water.

The high-concentration cell culture is performed using a fed-batch culture, specifically, a pH-stat feeding method or DO-stat feeding method, and a glucose solution at a concentration of 700g/L per 1L of the medium was supplied at 17.5 mL/h on average at the time when the glucose concentration in the culture medium became 0 g/L so that sugars which were added in the culture medium were consumed all by the microorganisms and the glucose concentration could be maintained at 0 g/L, and the optical density (OD) was measured using a UV-spectrometer to measure the cell concentration, and the $OD_{600}$ value was maintained in the range of 6 to 20. At 24 hours after starting the culturing, $OD_{600}$ reached approximately 150 (dry cell weight 50g/L).

After completing the high-concentration cell culture, the cell culture solution was used as it was without a separate recovery process in the subsequent step.

Comparative example 1-2. 3-HP production using high-concentration cultured cells (second step culturing)

A medium for producing 3-HP was prepared by adding 120g/L of glycerol and 10 $\mu$M vitamin B12 to 10mM phosphate buffer comprising no glucose. The cell culture solution prepared in Comparative example 1-1 was inoculated to the medium for producing 3-HP so that the cell inoculation amount was 10g/L (based on dry cell weight), and a 3-HP producing step was performed in a 5L fermenter (Working volume 2L).

As 3-HP production conditions, to maintain DO 5%, the stirring speed of the culture solution was set to 300 rpm, and aeration was sequentially adjusted by supplying at 1 vvm to form an aerobic condition. Other culture conditions were maintained at the temperature of 35°C and the pH of 6.8 using $Ca(OH)_2$ (Comparative example).

**Example 1. Preparation of 3-HP by fed-batch process and initial and additionally added glycerol control during 2-step cell culture for 3-HP production**

By applying a fed-batch process that performs the step of producing 3-HP by producing a high-concentration cell culture solution and then inoculating this to a production medium by the 2-step preparation method in the same manner as the method of Comparative example 1, but converting high-concentration 3-HP by dividing the addition amount of glycerol to overcome inhibition of production for high-concentration glycerol and consume maximal glycerol, an increase in 3-HP production resulting therefrom was confirmed.

Specifically, a medium for producing 3-HP was prepared so that the initial glycerol concentration could be 120g/L, and the step of producing 3-HP was performed under the condition in which glycerol corresponding to 100g/L was additionally supplied to the medium for producing 3-HP in a constant amount for 5 to 6 hours in 4 hours after the beginning of the culturing (Example).

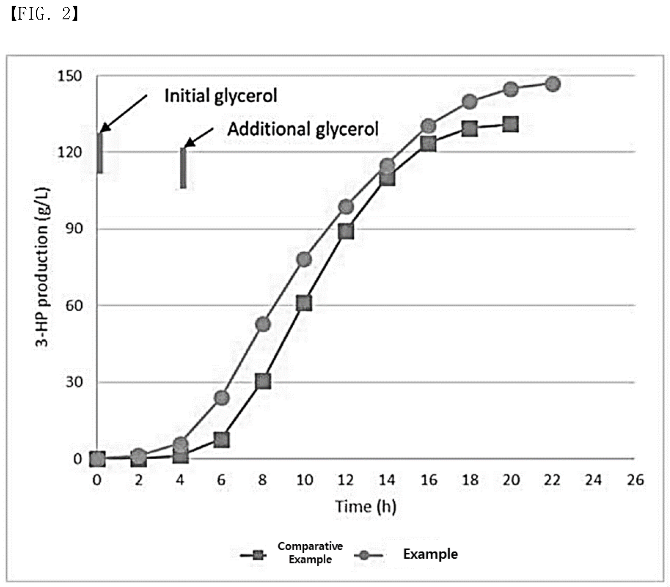

The concentration of the 3-HP produced according to the method for preparation of Comparative examples and Examples above was confirmed by HPLC analysis, and the results were shown in FIG. 2.

As shown in FIG. 2, when 3-HP was produced by the fed-batch process according to the present invention (Example), high-concentration 3-HP was produced at a higher level of 147g/L by overcoming inhibition of initial 3-HP production depending on the glycerol concentration to maintain cell activity at maximum compared to 130g/L of the case in which 3-HP was produced by the batch process (Comparative example), and in case of the 3-HP production ability measured by the following Equation 2 based on the corresponding production amount, it was produced with the productivity of 6.68 g/L/h higher than 6.55 g/L/h of the comparative example, and most of the added glycerol was consumed (remaining glycerol concentration of 1g/L or less), thereby minimizing the separation and purification load.

3-HP production ability = Final 3-HP production concentration (g/L) / Total 3-HP production time (h)　　　[Equation 2]

**Example 2. Comparison of 3-HP production amount depending on initial glycerol concentration during 3-HP production**

The production amount (g/L) of 3-HP depending on the initial glycerol concentration during 3-HP production was measured while producing 3-HP by the method of Example 1.

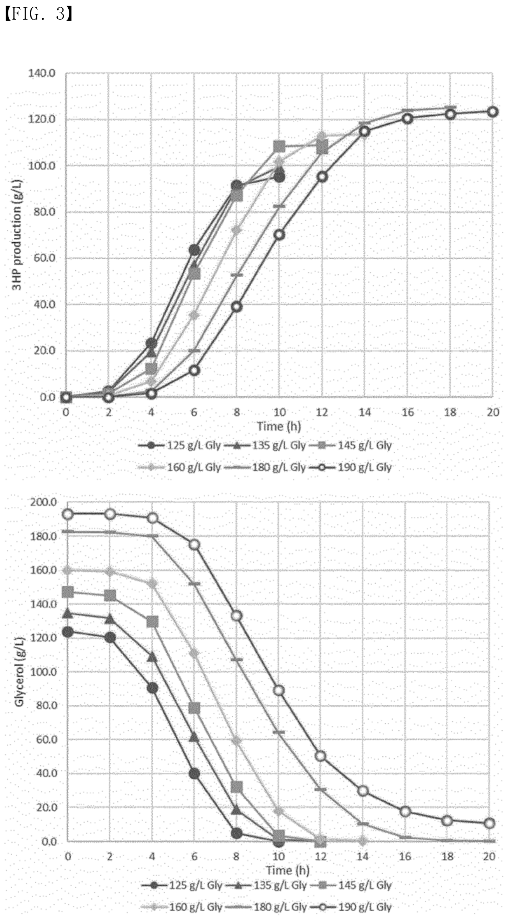

Specifically, the high-concentration cell culture and 3-HP production culture were performed by the method of Example 1, but the initial glycerol concentration in the 2-step 3-HP production culture medium was adjusted to 125, 135, 145, 160, 180 and 190 g/L, respectively, and the results of measuring the 3-HP production amount of the corresponding method were shown in FIG. 3.

As shown in FIG. 3, the production rate of 3-HP was inhibited as the concentration of glycerol was increased based on the glycerol concentration in the initial culture medium of 125g/L.

Under the glycerol concentration condition of 190g/L, which is higher than 180g/L, not all of the glycerol was consumed, and the production of 3-HP maintained at a level observed when the initial glycerol concentration was 120g/L.

In addition, under the glycerol condition of 125, 135 and 145 g/L, there were slight differences, but all of them showed a similar level of 3-HP productivity.

## Example 3. Fed-batch culture condition search for 3-HP optimal production

As similar 3-HP productivity was shown when the initial glycerol concentration was 125, 135 and 145g/L in Example 2, in order to confirm the production effect of 3-HP depending on the additionally supplied glycerol concentration according to the initial glycerol concentration condition lower or higher than the concentration range and the fed-batch process, the following experiment was performed.

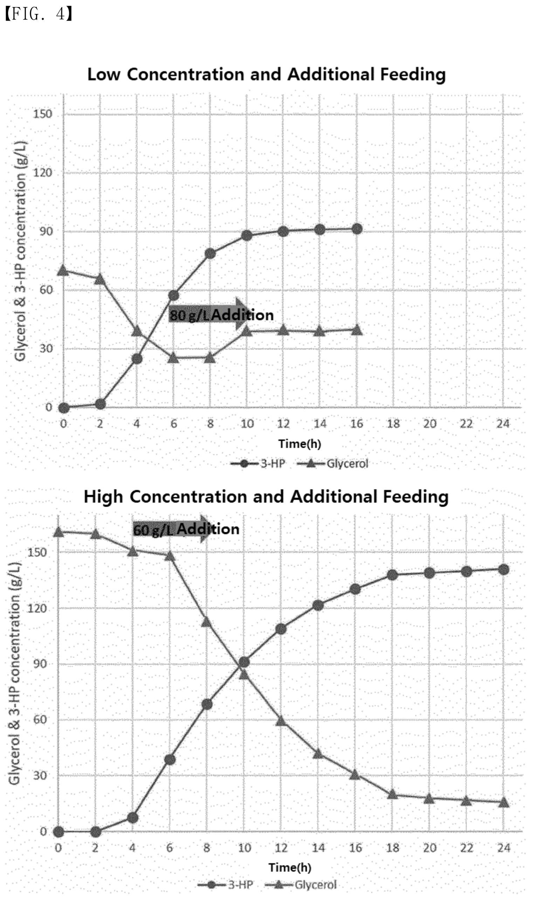

Specifically, 3-HP was produced according to Example 1 above, but under (i) the initial low-concentration glycerol condition (glycerol concentration: 70g/L) and the condition of adding glycerol of 80g/L in 6 hours after the beginning of production culture after that, and (ii) the initial high-concentration glycerol condition (glycerol concentration: 160g/L) and the condition of adding glycerol of 60g/L in 4 hours after the beginning of production culture, by the fed-batch process, 3-HP was produced, and the results were shown in FIG. 4.

As shown in FIG. 4, it was confirmed that the 3-HP production concentration was limited to 90g/L and thus it was not produced any more under the initial low-concentration glycerol condition and additional glycerol supply condition.

In addition, it was confirmed that 3-HP of 140g/L higher than 120g/L, which is the production concentration of the batch condition (Comparative example) was produced under the initial high-concentration glycerol condition and the additional supply condition, but it could be confirmed that glycerol of about 16g/L was not converted.

Based on these results, it can be seen that 3-HP of 147g/L can be optimally produced, as all of the glycerol was consumed, when the initial glycerol concentration is 120g/L as Example 1, and glycerol corresponding to 100g/L is additionally supplied during production culture.

## Claims

1. A method for producing 3-hydroxypropionic acid comprising the following steps:

   (1) high-concentration cell culturing strains having productivity of 3-hydroxypropionic acid (3-HP) in a culture medium; and
   (2) producing 3-HP by transferring the culture solution of the step (1) to a production medium comprising a substrate,
   wherein the producing 3-HP of the step (2) is performed by a fed-batch process, and
   the production medium comprises glycerol, and additional glycerol is added to the production medium during the production of 3-HP.

2. The method according to claim 1, wherein the cells having productivity of 3-HP comprises a gene encoding at least one protein selected from the group consisting of glycerol dehydratase and aldehyde dehydrogenase.

3. The method according to claim 1, wherein the culture medium of the step (1) does not comprise glycerol as a carbon source.

4. The method according to claim 3, wherein the carbon source is glucose.

5. The method according to claim 1, wherein the culture solution itself is transferred to the production medium without passing through an additional step including cell recovery, after the culturing of the step (1).

6. The method according to claim 1, wherein the concentration of glycerol in the production medium at the beginning of the culturing of the step (2) is 100 g/L to 150 g/L.

7. The method according to claim 1, wherein the addition amount of the additional glycerol is an amount corresponding to 70 to 140g/L based on the total volume of the production medium.

8. The method according to claim 1, wherein the addition of the additional glycerol is conducted for 5 to 6 hours.

9. The method according to claim 1, wherein the method has a productivity of 3-HP of 6 g/L/h or more.

10. The method according to claim 1, wherein the production amount of 3-HP of 100g/L to 1000g/L is shown during culturing for 20 to 30 hours.

11. The method according to claim 1, wherein the concentration in the total culture solution of remaining glycerol after producing is 2g/L or less.

12. A culture of a cell producing 3-hydroxypropionic acid, **characterized by** being prepared by the method for producing 3-hydroxypropionic acid according to any one of claim 1 to claim 11.

13. A composition for preparing 3-hydroxypropionic acid comprising the culture of claim 12.

14. The culture of a cell producing 3-hydroxypropionic acid according to claim 12, wherein the culture comprises 3-hydroxypropionic acid and orotic acid of 0.01 to 0.5 g/L as a by-product.

15. The culture of a cell producing 3-hydroxypropionic acid according to claim 14, wherein the culture of a cell producing comprises at least one by-product selected from the group consisting of acetic acid 0.01 to 1 g/L, propionic acid 0.01 to 0.5 g/L, succinic acid 0.01 to 1 g/L, formic acid 0.01 to 0.5 g/L, uracil acid 0.01 to 0.1 g/L, and citric acid 0.01 to 0.1 g/L.

16. The culture of a cell producing 3-hydroxypropionic acid according to claim 14 or claim 15, wherein the content of 3-hydroxypropionic acid in the culture is 100 g/L or more.

【FIG. 1】

Glycerol Feeding Strategy
[Adjustment of Input Amount, Timing, and Feeding Rate]

Conventional: Batch Process          New: Fed-batch Process

【FIG. 2】

【FIG. 3】

【FIG. 4】

Low Concentration and Additional Feeding

High Concentration and Additional Feeding

## INTERNATIONAL SEARCH REPORT

| International application No. |
|---|
| **PCT/KR2025/099165** |

| A. | CLASSIFICATION OF SUBJECT MATTER |
|---|---|

**C12P 7/52**(2006.01)i; **C12N 9/88**(2006.01)i; **C12N 9/02**(2006.01)i

According to International Patent Classification (IPC) or to both national classification and IPC

| B. | FIELDS SEARCHED |
|---|---|

Minimum documentation searched (classification system followed by classification symbols)

C12P 7/52(2006.01); C12N 1/16(2006.01); C12N 1/19(2006.01); C12N 15/70(2006.01); C12N 15/81(2006.01); C12P 7/42(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3-하이드록시프로피온산(3-hydroxypropionic acid), 생산(production), 배양 배지 (culture medium), 유가식 공정(fed-batch), 글리세롤(glycerol), 포도당(glucose)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2022-0015996 A (LG CHEM, LTD.) 08 February 2022 (2022-02-08)<br>See claims 1-4, 7 and 11-13; and paragraphs [0040], [0050], [0053] and [0098]. | 1-15 |
| Y | KR 10-2014-0085917 A (SAMSUNG ELECTRONICS CO., LTD.) 08 July 2014 (2014-07-08)<br>See claims 1, 3 and 4; and paragraphs [0009], [0031], [0036], [0037], [0039], [0041], [0058] and [0059]. | 1-15 |
| Y | KR 10-2013-0119945 A (NOVOZYMES, INC.) 01 November 2013 (2013-11-01)<br>See claim 36; and paragraph [0154]. | 14,15 |
| A | US 9365875 B2 (NOVOZYMES, INC.) 14 June 2016 (2016-06-14)<br>See claims 1 and 25. | 1-15 |
| A | KR 10-2011-0018118 A (SEOUL NATIONAL UNIVERSITY R&DB FOUNDATION et al.) 23 February 2011 (2011-02-23)<br>See claims 1 and 4. | 1-15 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **28 April 2025** | **29 April 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2025/099165**

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2022-0061044 A (LG CHEM, LTD.) 12 May 2022 (2022-05-12)<br>See claims 1 and 10. | 1-15 |

Form PCT/ISA/210 (second sheet) (July 2022)

**INTERNATIONAL SEARCH REPORT**

| International application No. |
| --- |
| **PCT/KR2025/099165** |

**Box No. II        Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **16**
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2025/099165**

| Patent document cited in search report | | | Publication date (day/month/year) | Patent family member(s) | | | Publication date (day/month/year) |
|---|---|---|---|---|---|---|---|
| KR | 10-2022-0015996 | A | 08 February 2022 | CN | 115956123 | A | 11 April 2023 |
| | | | | EP | 4190907 | A1 | 07 June 2023 |
| | | | | JP | 2023-531938 | A | 26 July 2023 |
| | | | | JP | 2024-163267 | A | 21 November 2024 |
| | | | | KR | 10-2639658 | B1 | 22 February 2024 |
| | | | | US | 2023-0265466 | A1 | 24 August 2023 |
| | | | | WO | 2022-025710 | A1 | 03 February 2022 |
| KR | 10-2014-0085917 | A | 08 July 2014 | KR | 10-1437042 | B1 | 02 September 2014 |
| KR | 10-2013-0119945 | A | 01 November 2013 | AU | 2011-336923 | A1 | 30 May 2013 |
| | | | | AU | 2011-336923 | B2 | 16 February 2017 |
| | | | | BR | 112013012630 | A2 | 24 September 2020 |
| | | | | BR | 112013012630 | B1 | 08 September 2021 |
| | | | | CA | 2818499 | A1 | 07 June 2012 |
| | | | | CN | 103502432 | A | 08 January 2014 |
| | | | | CN | 107828671 | A | 23 March 2018 |
| | | | | CN | 107828671 | B | 08 March 2022 |
| | | | | EP | 2643450 | A2 | 02 October 2013 |
| | | | | EP | 3287519 | A1 | 28 February 2018 |
| | | | | EP | 3287519 | B1 | 18 March 2020 |
| | | | | JP | 2013-542747 | A | 28 November 2013 |
| | | | | JP | 6061862 | B2 | 18 January 2017 |
| | | | | MX | 2013005654 | A | 17 July 2013 |
| | | | | US | 10260072 | B2 | 16 April 2019 |
| | | | | US | 10633664 | B2 | 28 April 2020 |
| | | | | US | 11118187 | B2 | 14 September 2021 |
| | | | | US | 12054721 | B2 | 06 August 2024 |
| | | | | US | 2012-0135481 | A1 | 31 May 2012 |
| | | | | US | 2014-0065681 | A1 | 06 March 2014 |
| | | | | US | 2016-0177317 | A1 | 23 June 2016 |
| | | | | US | 2018-0044684 | A1 | 15 February 2018 |
| | | | | US | 2019-0249181 | A1 | 15 August 2019 |
| | | | | US | 2020-0291408 | A1 | 17 September 2020 |
| | | | | US | 2022-0064654 | A1 | 03 March 2022 |
| | | | | US | 9090918 | B2 | 28 July 2015 |
| | | | | US | 9777280 | B2 | 03 October 2017 |
| | | | | WO | 2012-074818 | A2 | 07 June 2012 |
| | | | | WO | 2012-074818 | A3 | 27 September 2012 |
| | | | | ZA | 201303544 | B | 26 November 2014 |
| US | 9365875 | B2 | 14 June 2016 | AU | 2013-352418 | A1 | 28 May 2015 |
| | | | | AU | 2013-352418 | B2 | 08 December 2016 |
| | | | | BR | 112015012409 | A2 | 12 September 2017 |
| | | | | CA | 2891130 | A1 | 05 June 2014 |
| | | | | CN | 105209626 | A | 30 December 2015 |
| | | | | CN | 105209626 | B | 03 September 2019 |
| | | | | EP | 2925873 | A1 | 07 October 2015 |
| | | | | EP | 2925873 | B1 | 12 July 2017 |
| | | | | JP | 2015-536669 | A | 24 December 2015 |
| | | | | US | 2014-0154760 | A1 | 05 June 2014 |
| | | | | WO | 2014-085330 | A1 | 05 June 2014 |
| KR | 10-2011-0018118 | A | 23 February 2011 | KR | 10-1048151 | B1 | 11 July 2011 |

Form PCT/ISA/210 (patent family annex) (July 2022)

**INTERNATIONAL SEARCH REPORT**
**Information on patent family members**

International application No.

**PCT/KR2025/099165**

| Patent document cited in search report | | | Publication date (day/month/year) | Patent family member(s) | | | Publication date (day/month/year) |
|---|---|---|---|---|---|---|---|
| KR | 10-2022-0061044 | A | 12 May 2022 | CN | 116323957 | A | 23 June 2023 |
| | | | | EP | 4242319 | A1 | 13 September 2023 |
| | | | | KR | 10-2696014 | B1 | 16 August 2024 |
| | | | | US | 2023-0340547 | A1 | 26 October 2023 |
| | | | | WO | 2022-098162 | A1 | 12 May 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240015266

- KR 1020210151641